# EUROPEAN PATENT SPECIFICATION

(11) **EP 3 188 596 B1**
(45) Date of publication and mention of the grant of the patent: **28.10.2020**
(21) Application number: 15775805.3
(22) Date of filing: 02.09.2015
(51) Int. Cl.: A01N 1/00, A61L 27/60, A61L 27/36

(54) **HUMAN DERMIS, PREPARATION AND USE THEREOF**
MENSCHLICHE DERMIS, HERSTELLUNG UND VERWENDUNG DAVON
DERME HUMAIN, PRÉPARATION ET UTILISATION

(30) Priority: 02.09.2014 IT FI20140197
(43) Date of publication of application: 12.07.2017
(73) Proprietor: Azienda Ospedaliera Universitaria Senese, 53100 Siena (IT); Tissuelab S.r.l. Con Unico Socio, 50132 Firenze (IT)
(72) Inventor: PIANIGIANI, Elisa, I-53100 Siena (IT); RAMELLI, Massimiliano, I-81100 Caserta (IT); DELLA VALLE, Elena, I-81100 Caserta (IT)
(74) Representative: Valenza, Silvia
(86) International application number: PCT/IB2015/056658
(87) International publication number: WO 2016/035019

(56) References cited:
- WO-A2-2008/154623
- WO-A2-2009/050571
- US-A1- 2003 035 843
- US-B1- 8 735 054
- ROONEY P ET AL: "Sterilisation of skin allograft with gamma irradiation", BURNS, BUTTERWORTH HEINEMANN, GB, vol. 34, no. 5, 1 August 2008 (2008-08-01) , pages 664-673, XP022708140, ISSN: 0305-4179, DOI: 10.1016/J.BURNS.2007.08.021 [retrieved on 2008-01-15]

## Description

### Field of the invention

The present invention relates to the field of connective tissues treated for applications in regenerative medicine.

### Prior art

The main problem in the therapy of full-thickness cutaneous substance losses is the need to reconstruct a dermis expressing the biological and physical features which are typical of the anatomic structure lost.

In order to cope with and overcome this need, different methods have been developed in recent decades which use human deepidermized dermis (DED) for reconstructing the dermal part in full-thickness substance losses followed by grafting keratinocyte suspensions or sheets obtained *in vitro* for the epithelial component integration. A culture epidermis has thus been obtained with features similar to that *in vivo* (Berthod F, Damour O.: In vitro reconstructed skin models for wound coverage in deep burns. Br. J. Dermatol. 1997; 136: 809-816).

This transplantation technique devised by Cuono et al. (Cuono CB et al.: Composite autologous-allogeneic skin replacement: development and clinical application. Plast Recontr Surg.1987; 80: 626) has met a certain consensus in the treatment of severe burns, where the dermis is derived from allogenic skin and the epidermis from autologous keratinocyte cultures.

Several studies have shown that dermis allografts, obtained by removing the epidermis from allogenic skin, represent the ideal substrate for the integration of autologous cell cultures or thin autografts in order to obtain the healing of cutaneous substance loss. In fact, the poor immunogenicity of these allografts ensures good engraftment and quick colonization by the host cells, thus preventing both the graft contraction and the repair with scar tissue; this ultimately promotes the "physiologic" wound repair.

The first allograft model of deepidermized dermis, devised by Pruniéras at al. (Pruniéras M, Régnier M, Woodley D. Methods for cultivation of keratinocytes with an air-liquid interface. J Invest Dermatol. 1983 Jul;81-1 Suppl: 28s-33s) used as substrate DED, obtained by deepidermizing cadaver skin subjected to irradiation or successive freezing and thawing.

A commercial example of human acellular dermis from cadaver skin is given by a product called AlloDerm®, where the dermis, processed under aseptic conditions, obtained by deepidermizing the skin, is treated with a stabilizing solution, a decellularizing solution (0.5% anionic detergent), a cryoprotective solution in sequence, and finally freeze-dried.

The product is used in the clinical field, both in the treatment of deep partial or full-thickness wounds in burnt patients and in the repair or replacement of damaged soft tissues, supporting the tissue regeneration of the host, and promoting the revascularization processes.

Another dermal substitute available on the market is DermaMatrix Acellular Dermis®, produced by MTF and distributed by Synthes, Inc.

The starting raw material is, as with AlloDerm, deepidermized dermis, which is decellularized through a series of washes with detergents and acids, freeze-dried and sterilized. It is used in the surgical field for the reconstruction of cutaneous substance losses.

LifeNet Health is also available on the market with ArthroFlex®, an acellular dermal matrix available in the surgical field for supporting and repairing soft tissues. For this product, the decellularization process comprises isolating a tissue sample from a suitable donor, treating the sample with a weakly alkaline, buffered hypotonic endonuclease solution, with an anionic detergent in addition to a possible hypertonic saline buffer solution. The tissue is then washed with water and with an aqueous solution of chlorine dioxide and then stored in a sealed package in the presence of an isotonic or chlorine dioxide or isopropanol 70% solution.

A further method for obtaining acellularized dermis from the skin of a cadaver donor (WO 2009050571, 23/04/2009) is to treat the deepidermized dermis with an enzyme solution, followed by the irradiation with ionizing electromagnetic radiation (gamma radiation with a frequency between 10¹⁹÷10²² Hz, beta radiation or a combination thereof) in order to obtain a sterilized acellular tissue with structurally intact extracellular matrix to be used as a support for the engraftment of both autologous and homologous stem cells; in this patent, the freeze-drying step is not included.

It is apparent, in the light of the above, that the availability of a homologous product in the field of skin regeneration is required, which as such allows to prevent the morbidity problems associated with a similar biologically safe autologous product which does not cause immune response by the host, does not require special storage conditions, and carrying out its support function, allows the dermal tissue to be quickly restored anatomically and functionally.

### Summary of the invention

There is described the preparation of deepidermized human dermis, which is then decellularized, freeze-dried and sterilized.

### Detailed description of the invention

The present invention relates to a process for obtaining deepidermized (DED) human dermis, where said dermis is decellularized, freeze-dried and sterilized. The dermis thus obtained retains the extracellular protein matrix, while eliminating the cellular component thereof which is responsible for immune or rejection reactions.

The process according to the invention uses deepidermized dermis, DED, from homologous skin from cadaver donor (allograft).

The tissue collection from cadaver donor is carried out according to the known methods and occurs only in the operating room with consequent minimization of the initial (pre-processing sample) and final (post-processing sample) bioburden on the tissue. The collection is carried out by appropriately trained physicians and exactly performed as surgery. All this justifies the data (Processing efficacy in relation to microbial contamination of skin allografts from 723 donors. Burns. 2010 May; 36(3):347-51) of a contamination of the pre-processing tissue with rare colonies, in approximately ¼ of the donors, with a further reduction of such a contamination during processing at the bank, which leads to the final result of a tissue contamination at the end of the processing at the bank (before freeze-drying and gamma-irradiation) of 1.24% (with few colonies).

In parallel, the microbiological tests carried out are very accurate and are based on research of aerobic/anaerobic bacteria and fast/medium/slow growth fungi, with incubation times of as much as 3 weeks.

The cadaver donors are tested in relation to transmissible diseases as set forth by the Guidelines of the National Transplant Centre (year 2013) and analyzed for: HIV1/2Ab; HCVAb; HBcAb; HBsAb, HbsAg; CMV IgG and IgM; HTLV-I/IIAb; TPHA or other test which detects treponema antibodies. In addition to the serological tests above, donors are subjected to the following biomolecular tests: HIV ½ NAT; HBV NAT; HCV NAT. The use of these tests involves a wide margin of safety of the allograft for clinical use, thus greatly reducing the window period of the three human viruses above.

In parallel, the microbiological tests carried out are very accurate and are based on research of aerobic/anaerobic bacteria and fast/medium/slow growth fungi, with incubation times of as much as 3 weeks.

The collected dermis is first deepidermized according to known methodologies and it is then subjected to 2 treatments, normally 20-40 minutes each, with a hypotonic solution, in which it is completely immersed, which takes place under stirring (180rpm÷200rpm) at 30°C-40°C. At the end of the first treatment, the solution is eliminated by pouring and replaced with a new solution to proceed with the second treatment.

Thereafter, the DED is incubated with a zwitterionic detergent at predetermined concentration and time. Two incubations are preferably performed with CHAPS [(3-[(3-Cholamidopropyl) dimethylammonium]-1-propanesulfonate)], the molarity being from 0.01M to 0.03M, the concentration of 0.02M being particularly preferred, leaving the tissue in contact with the detergent for 70 -110 minutes in a sonicator (30Hz÷50Hz), preferably at 30°C-40°C in the first incubation and overnight in a thermostatic bath under stirring (180rpm÷200rpm) in the second incubation, preferably at 30°C-40°C.

At least one wash (5-30 minutes) is carried out with purified water (WFI) at 30°C-40°C, preferably in a sonicator at 37°C for 10 minutes.

The wash is also effective in the removal of detergent residues, determining excellent compatibility which is shown by a rapid adhesion of cells to the fibrous structure and resulting good ability of recolonization of the dermal matrix thus treated, both by normal human fibroblasts in culture and by HaCaT line keratinocyte (ref.: Siena Study 11/05/2015; Siena Report 13/06/2013).

At least 2 treatments of the tissue are then carried out (the first of 60-120 minutes, the second of 10-60 minutes) with a buffer solution at neutral pH; for example, 500 mM monobasic phosphate buffer pH 7.0. Of the 2 treatments, the first takes place in a sonicator at 30°C-40°C and the second under stirring (180rpm÷200rpm) at 30°C-40°C. Finally, at least one wash is carried out (20-40 minutes) with purified water under stirring (180rpm÷200rpm) at 30°C-40°C.

The DED is then treated with the radioprotective solution Clearant® consisting of DMSO, Propylene glycol, Trehalose, Mannitol (4 hours) at 40°C under stirring (180rpm÷200rpm).

The tissue is freeze-dried after being suitably shaped and packaged into freeze-drying bags, such as Tyvek® (a DuPont registered material consisting of high-density polyethylene fibers) so as to ensure a residual moisture content of less than 15% and minimize the stress associated with the freeze-drying step and damage at the end of the entire process. This process also ensures that storage standards are retained for five years.

After the freeze-drying, each tyvek/polypropylene bag containing the tissue is sealed under vacuum in an aluminum/polypropylene bag.

Thereafter, the bags thus prepared are accommodated inside a heat-insulated container of foamed polystyrene, interspersed in the accommodation with dry ice, and are subjected to a sterilization treatment with gamma rays emitted by a Cobalt 60 source. The ionizing dose normally used is of 25kGy÷35kGy, and during the treatment, the temperature is maintained at -80°C.

After the bacterial sterilization, tissues are stored at room temperature (15°C-30°C) until use.

The process as described differs from the prior art in the combination of the effects of detergent and sonication, which make the decellularization step particularly effective in the conditions identified. Moreover, the cryoprotective and radioprotective solution preserves the ultrastructure of the collagen fibers and the integrity of the elastic fibers differently from tissues gamma-irradiated in the absence of protective solution.

An advantageous aspect of the present invention is obtaining histocompatible dermis, given the high decellularization level of the product. Another advantage of the invention is that it allows a decellularized, freeze-dried and sterilized dermis to be obtained, which retains the structure of the dermal extracellular matrix with its fibrous structure, collagen and elastic. A further advantageous aspect of the invention is the possibility of storing the dermis at room temperature, thanks to the end freeze-drying and sterilization process to which it is subjected, which ensures a safe level of sterility (SAL: Sterility Assurance Level) of 10⁻⁶.

The present invention therefore also relates to the dermis obtained according to the process described above.

The dermis thus treated can be used as a skin graft in the replacement or repair therapy of damaged or unsuitable tegumentary tissue, or in the regenerative therapy using the dermal matrix as a filler for reconstructions or as a scaffold in orthopedic or dentistry surgery.

The invention will now be better illustrated in the light of the specific example set forth below.

As already mentioned, the first step of processing the skin collected from cadavers as described above, that is, the elimination of the epidermal component, is carried out according to known methods to obtain the deepidermized dermis DED, and was performed at the Centro Conservazione Cute-AOUSenese in a classified laboratory (Class B - GMP), accredited by the National Transplant Center (year 2005) and considered a tissue bank of national interest.

The second processing step, i.e. decellularization, freeze-drying and sterilization (to which the examples below specifically refer) was performed at Tissuelab (Class C - GMP).

### Example

The homologous deepidermized dermis from donation, 29 patches for a total area of about 300cm² and a thickness from 200µm to 800µm, was subjected to cell lysis through 2 consecutive treatments with purified water. The DED, transferred to a sterile container (Thermo Scientific Nalgene 2 liters, model 2121-0005), was completely immersed in purified water and incubated (thermostatic bath Julabo model SW23) under stirring at 190rpm for 30 minutes at 37°C. At the end of the first treatment, water was removed from each container by pouring, other purified water was added and the DED was incubated again for 30 minutes at 37°C at 190rpm.

In the next step the DED, from which the lysis water was removed, was treated 2 consecutive times with the CHAPS [(3-[(3-Cholamidopropyl) dimethylammonium]-1-propanesulfonate)] 0.02M detergent.

During the first treatment, the DED was put in contact with the detergent for 90 minutes at 37°C in a ultrasonic bath (sonicator Soltec - Sonica model EP 3300), the detergent solution was then removed and the second treatment was carried out, where the DED was incubated in a thermostatic bath with the detergent at the temperature of 37°C at 190rpm overnight.

In order to remove the detergent and the cell debris, the DED was treated with purified water in an ultrasound bath at 37°C for 10 minutes, followed by two treatments in sequence with 500mM monobasic phosphate buffer solution pH 7.0, the first of which in ultrasound bath for 90 minutes at 37°C and the second in a thermostatic bath under stirring at 190rpm for 30 minutes at 37°C, and the last treatment carried out by washing the DED with purified water for 30 minutes at 37°C in a thermostatic bath at 190rpm.

Then, the DED was treated with radioprotective solution (CRP solution - Clearant®) for 4 hours in a thermostatic bath at a temperature of 40°C under stirring at 120rpm.

At the end, the DED was laid down on sterile membrane (N-Terface®) and covered with the same, then it was shaped into patches with precise dimensions. Altogether, 36 1x2 cm patches, 17 2x2cm patches, 8 2x5cm patches were obtained. Each shaped patch was transferred into a Tyvek/polypropylene bag and subjected to the freeze-drying cycle (freeze-drier Virtis model Genesis 25 Super XL).

At the end of the freeze-drying cycle, the residual moisture was found to be less than 15% (coulometric method).

In this example, the freeze-drying process was conducted according to the following schedule:
1. Freezing step: Plate temperature -30°C, 90 minutes.
2. Primary drying step: Once the predetermined temperature and pressure conditions (plate temperature -30°C, condenser temperature -40°C, chamber pressure 500mT) have been reached, in a time period of 60 minutes the pressure is reduced to 100mT and the plate temperature is increased to +5°C. Such pressure and temperature values, 100mT and +5°C, were maintained for 480 minutes. At the end, the sample storage conditions were achieved, reducing the plate temperature to -10°C and increasing the pressure to 200mT.

The Tyvek/propylene bags containing the freeze-dried DED patches were closed and sealed under vacuum in aluminum/polypropylene bags.

At the end of the packaging, the freeze-dried DED was stored at -80°C until the sterilization treatment, carried out using an ionizing dose of 25kGy.

The freeze-dried and sterilized DED were stored at room temperature (15°C÷30°C).

Freeze-dried and sterilized samples collected both at the end of the process just described and at the end of other 2 independent processing operations, performed similarly to the Example herein, were used to conduct *in vitro* tests in order to evaluate the cytotoxicity of the same. The results of tests (MTT assay) showed that the freeze-dried and sterilized DED had no cytotoxic effects; in fact, at the end of the tests no reduction in the cell vitality of more than 30% was observed (Cytotoxic Effect Limit: Vitality Reduction > 30%).

Moreover, histological tests were carried out on similar samples of lyophilized and sterilized DED in order to evaluate the effectiveness of the process described both for the purpose of tissue decellularization and for the purpose of retaining the integrity of the dermal matrix. At the optical microscopy, the morpho-structural analysis of the preparations showed a good preservation of the fibrous structure (collagen and elastic), but it showed the persistence of some cells within the vascular structures and the annexes and thus, not a complete decellularization. The deepidermization was complete and the basal membrane retention was good. On the other hand, the electron microscopy showed: absence of epidermis, recognizable basal membrane, collagen fibers, in which the period can be documented, properly structured and conserved, clearly visible elastic component. No vascular structures or nerve endings were observed and few degenerated cells, presumably fibroblasts, were found. In summary, the EM showed the retention of the structure of the dermal extracellular matrix with collagen and elastic present and substantially normal with the presence of rare degenerated cellular elements.

Finally, during the processing described in Example 1 and in 2 other identical independent processing operations, checks were carried out to evaluate the mechanical properties of the deepidermized dermis after sterilization by gamma radiation. In detail, samples were collected both after freeze-drying and after sterilization. The study evaluated the following: the elastic moduli Emin and Emax, expressed in MPa; the maximum stress endured by the material (σmax), expressed in MPa, and the breaking strain (εr), expressed as a percentage, at the rupture of the sample. All the samples in the study, except for one freeze-dried and not sterilized, showed a breaking strain greater than 80%. The elastic moduli Emin and Emax obtained from the stress curve with respect to deformation showed superimposable patterns for the non sterilized samples compared to the sterilized ones. In conclusion, the analyzed samples showed no alteration of the mechanical abilities resulting from the sterilization treatment, confirming the results obtained in the histological tests which showed the integrity of the elastic fibers of the treated tissues.

In order to evaluate the engraftment capacity and the ability of the deepidermized, decellularized, freeze-dried and sterilized dermis in guiding the tissue repair and regeneration process, a preliminary comparison study was conducted at AOUSenese (in press) on n. 8 patients suffering from oral cavity cancers, after appropriate informed consent. The loss of full thickness substance, upon the removal of the tumor, was repaired by means of grafts (2-4 cm²) of deepidermized, decellularized, freeze-dried and sterilized dermis directly sutured into the surgical wound after reconstitution in saline. In nearly all cases (7/8 patients), engraftment and recolonization and revascularization of the graft was observed in a short time (about 3 weeks).

The odontostomatology surgeon users have used, for comparison in similar cases, various types of membranes (Goretex synthetic, biological of human and bovine collagen, equine pericardium), all for internal use, i.e. used for regenerative purpose, usually for the guided regeneration of bones or tissue (Guided Bone/Tissue Regeneration, GBR/GTR): these membranes have as a prerequisite the need to be "covered" by the mucous membrane as their exposure leads, generally, to the failure of the regenerative process. The use of the decellularized, freeze-dried, irradiated DED showed an excellent engraftment ability (1/8 cases of graft detachment) with the absolutely clinically interesting possibility of exposing the membrane directly in the oral environment, without the graft being reabsorbed. From the ongoing study (which will continue in the future), the present bioproduct is a graft of excellent quality for oral reconstructive surgery, which is better than the products presently used, with key features such as: good handling, excellent resistance, good adhesion. After the engraftment, the healing is surprisingly easy and quick.

## Claims

1. A process for treating human dermis, wherein a human dermis collected from a cadaver and deprived of the epidermal component is:
(a) decellularized;
(b) freeze-dried;
(c) sterilized;
**characterized in that** the deepidermized dermis is decellularized (a) as follows:
- two consecutive treatments in purified water of 20'-40' each at 30°-40°C under stirring;
- two incubations with a zwitterionic detergent solution in a concentration of 0.03-0.01 M, the first of which lasting 70'-110' in an ultrasonic bath at 30°-40°C and the second being overnight under stirring in a thermostatic bath again at 30°-40°C;
- a wash with purified water at 30°-40°C for 5-30 minutes in an ultrasonic bath and two washes with buffer at neutral pH, the first of which occurring in a sonicator at 30°-40°C and the second in a thermostatic bath under stirring at 30°-40°C, followed by at least one wash of 20'-40' with purified water under stirring at 30°-40°C.

2. A process according to claim 1, wherein the dermis obtained by the treatment according to claim 1:
- is treated in a radioprotective solution consisting of DMSO, Propylene glycol, Trehalose, Mannitol for 4 hours at 37°C,
- the shaped edges are packaged inside bags made of a material consisting of high-density polyethylene fibers;
- the bagged dermis is freeze-dried as follows:
- the freezing step lasts 90' and the temperature of the inner plate of the freeze drier is of -30°C;
- the primary drying step starts when the plate temperature is -30°C, the condenser temperature is -40°C, the chamber pressure is of 500mT and in a time period of 60' the pressure is reduced to 100mT and the plate temperature is increased to +5°C and such pressure and temperature values are maintained for 480 minutes;
- at the end of the primary drying step, the sample storage conditions are achieved, reducing the plate temperature to -10°C and increasing the pressure to 200mT.

3. A process according to claim 1, wherein the dermis obtained according to claim 2 is sterilized by means of gamma rays emitted by a Cobalt 60 source, at an ionizing dose of 25kGy and at the temperature of -80 °C.

4. A human dermis which is deepidermized, decellularized, freeze-dried and sterilized according to the process of claims 1-3.

5. A human dermis according to claim 4 for use as a skin graft in the replacement or repair therapy of damaged or unsuitable tegumentary tissue, or in the regenerative therapy using the dermal matrix as a filler for reconstructions or as a scaffold in orthopedic or dentistry surgery.

6. The human dermis for use according to claim 5, wherein said deepidermized, decellularized, freeze-dried and sterilized dermis is previously rehydrated in saline for 1 hour at room temperature or for 30' at +37°C.

## Patentansprüche

1. Verfahren zur Behandlung von humaner Dermis, wobei eine humane Dermis, die von einem Kadaver entnommen und der epidermalen Komponente entbehrt wird:
(a) dezellularisiert;
(b) gefriergetrocknet;
(c) sterilisiert wird;
**dadurch gekennzeichnet, dass** die deepidermisierte Dermis (a) wie folgt dezellularisiert wird:
- zwei aufeinanderfolgende Behandlungen in Reinstwasser für jeweils 20'-40' bei 30°-40°C unter Rühren;
- zwei Inkubationen mit einer zwitterionischen Detergenslösung in einer Konzentration von 0,03-0,01 M, von denen die erste 70'-110' in einem Ultraschallbad bei 30°-40 °C dauert und die zweite über Nacht unter Rühren in einem thermostatischen Bad wiederum bei 30°-40 °C erfolgt;
- eine Wäsche mit Reinstwasser bei 30°-40 °C für 5-30 Minuten in einem Ultraschallbad und zwei Wäschen mit Puffer bei neutralem pH-Wert, von denen die erste in einem Ultraschallgerät bei 30°-40 °C und die zweite in einem thermostatischen Bad unter Rühren bei 30°-40 °C erfolgt, gefolgt von mindestens einer Wäsche für 20'-40' mit Reinstwasser unter Rühren bei 30°-40 °C.

2. Verfahren gemäß Anspruch 1, wobei die durch die Behandlung gemäß Anspruch 1 erhaltene Dermis:
- in einer Strahlenschutzlösung, bestehend aus DMSO, Propylenglykol, Trehalose, Mannitol, für 4 Stunden bei 37 °C behandelt wird,
- die geformten Ränder in Beuteln aus einem Material verpackt werden, das aus hochdichten Polyethylenfasern besteht;
- die in Beutel verpackte Dermis wie folgt gefriergetrocknet wird:
- der Gefrierschritt 90' dauert und die Temperatur der Innenplatte des Gefriertrockners -30 °C beträgt;
- der primäre Trocknungsschritt beginnt, wenn die Plattentemperatur -30 °C beträgt, die Temperatur des Kondensators -40 °C beträgt, der Kammerdruck 500 mT beträgt und in einer Zeitspanne von 60' der Druck auf 100 mT reduziert und die Plattentemperatur auf +5 °C erhöht wird und diese Druck- und Temperaturwerte für 480 Minuten aufrechterhalten werden;
- am Ende des primären Trocknungsschritts die Bedingungen für die Probenaufbewahrung erreicht werden, wobei die Plattentemperatur auf
- 10 °C reduziert und der Druck auf 200 mT erhöht wird.

3. Verfahren gemäß Anspruch 1, wobei die gemäß Anspruch 2 erhaltene Dermis mit Hilfe von Gammastrahlen, die von einer Kobalt-60-Quelle emittiert werden, bei einer ionisierenden Dosis von 25 kGy und bei einer Temperatur von -80 °C sterilisiert wird.

4. Humane Dermis, die nach dem Verfahren gemäß Anspruch 1-3 deepidermisiert, dezellularisiert, gefriergetrocknet und sterilisiert wird.

5. Humane Dermis gemäß Anspruch 4 zur Verwendung als Hauttransplantat bei der Ersatz- oder Reparaturtherapie von beschädigtem oder ungeeignetem tegumentären Gewebe oder bei der regenerativen Therapie unter Verwendung der Matrix der Dermis als Füllstoff für Rekonstruktionen oder als Gerüst in der orthopädischen oder zahnärztlichen Chirurgie.

6. Humane Dermis zur Verwendung gemäß Anspruch 5, wobei die deepidermisierte, dezellularisierte, gefriergetrocknete und sterilisierte Dermis zuvor für 1 Stunde bei Raumtemperatur oder für 30' bei +37 °C in Kochsalzlösung rehydriert wird.

## Revendications

1. Un procédé pour traiter du derme humain, dans lequel un derme humain collecté à partir d'un cadavre et dépourvu du composant épidermique est :
(a) décellularisé ;
(b) lyophilisé ;
(c) stérilisé ;
**caractérisé en ce que** le derme dé-épidermisé est décellularisé (a) comme suit :
- deux traitements consécutifs dans de l'eau purifiée de 20' à 40' chacun de 30 ° à 40 °C sous agitation ;
- deux incubations avec une solution de détergent zwitterionique dans une concentration de 0,03 à 0,01 M, la première durant de 70' à 110' dans un bain à ultrasons de 30° à 40 °C et la deuxième durant une nuit sous agitation dans un bain thermostatique à nouveau de 30° à 40 °C ;
- un lavage avec de l'eau purifiée de 30° à 40 °C pendant 5 à 30 minutes dans un bain à ultrasons et deux lavages avec tampon à pH neutre, le premier se produisant dans un sonicateur de 30° à 40 °C et le deuxième dans un bain thermostatique sous agitation de 30° à 40 °C, suivi d'au moins un lavage de 20' à 40' avec de l'eau purifiée sous agitation de 30° à 40 °C.

2. Un procédé selon la revendication 1, dans lequel le derme obtenu par le traitement selon la revendication 1 :
- est traité dans une solution radioprotectrice constituée de DMSO, Propylène glycol, Tréhalose, Mannitol pendant 4 heures à 37 °C,
- les bords façonnés sont conditionnés à l'intérieur de sacs faits d'un matériau constitué de fibres de polyéthylène haute densité ;
- le derme ensaché est lyophilisé comme suit :
- l'étape de congélation dure 90' et la température de la plaque interne du lyophilisateur est de -30 °C ;
- l'étape de séchage primaire commence lorsque la température de la plaque est de -30 °C, la température du condenseur est de -40 °C, la pression de la chambre est de 500 mT et dans une période de temps de 60' la pression est réduite à 100 mT et la température de la plaque est augmentée à +5 °C et ces valeurs de pression et de température sont maintenues pendant 480 minutes ;
- à la fin de l'étape de séchage primaire, les conditions de stockage d'échantillon sont obtenues, réduisant la température de la plaque à -10 °C et augmentant la pression à 200 mT.

3. Un procédé selon la revendication 1, dans lequel le derme obtenu selon la revendication 2 est stérilisé au moyen de rayons gamma émis par une source de Cobalt 60, à une dose d'ionisation de 25 kGy et à la température de -80 °C.

4. Un derme humain qui est dé-épidermisé, décellularisé, lyophilisé et stérilisé selon le procédé des revendications 1 à 3.

5. Un derme humain selon la revendication 4 destiné à être utilisé en tant que greffe cutanée dans la thérapie de remplacement ou de réparation de tissu de tégument endommagé ou inapproprié, ou dans la thérapie régénérative à l'aide de la matrice dermique en tant que charge pour des reconstructions ou en tant qu'échafaudage en chirurgie orthopédique ou dentaire.

6. Le derme humain destiné à être utilisé selon la revendication 5, ledit derme dé-épidermisé, décellularisé, lyophilisé et stérilisé étant préalablement réhydraté en solution saline pendant 1 heure à température ambiante ou pendant 30' à +37 °C.
